# EUROPEAN PATENT SPECIFICATION

(11) **EP 0 445 915 B1**
(45) Date of publication and mention of the grant of the patent: **09.08.1995**
(21) Application number: 91300787.8
(22) Date of filing: 31.01.1991
(51) Int. Cl.: C07K 1/04, B01J 19/00

(54) **Method of performing a multiple synthesis of peptides on a solid carrier and apparatus for carrying out this method**
Verfahren zur Ausführung einer vielfachen Synthese von Peptiden auf einem festen Träger und Vorrichtung zur Durchführung dieses Verfahrens
Méthode pour effectuer la synthèse de plusieurs peptides sur un support solide et appareillage pour exécuter cette méthode

(30) Priority: 02.02.1990 CS 508/90
(43) Date of publication of application: 11.09.1991
(73) Proprietor: AKADEMIE VED CESKE REPUBLIKY, Praha 1 (CZ)
(72) Inventor: Lebl, Michal, Prague (CS); Eichler, Jutta, Berlin (DE); Pokorny, Vit, Prague (CS); Jehnicka, Jiri, Prague (CS); Mudra, Petr, Prague (CS); Zenisek, Krel, Prague (CS); Stierandova, RNDR Alena, Cerveny Kostelec (CS); Kalousek, Jan, Prague (CS); Bolf, Jan, Roztoky (CS)
(74) Representative: Enskat, Michael Antony Frank

(56) References cited:
- DE-A- 3 723 004
- Tetrahedron Vol. 44, No. 19 pp. 6031-6040, 1988
- Innovation and perspectives in solid phase syntheses, collected papers of the internat. symp. 1989, pages 337-343; 2,

## Description

The invention relates to a method and device for carrying out a multiple synthesis on a solid carrier. The synthesis technology of peptides has been developed from conventional methods used for a synthesis carried out in a solution (the survey is mentioned in Houben-Weyl Methoden der organischen Chemie, Synthese von Peptiden, E Wunsch ed, Thieme, Berlin 1974), through the synthesis technique developed by Merrifield applying a solid carrier in the form of particles (as to the survey of the present state, see eg Stewart J M and Young J D, Solid Phase Peptide Synthesis, Freeman, San Francisco 1985) which was suitable for automation (see eg Merrifield R B, Stewart J M and Jernberg N, Apparatus for the Automated Synthesis of Peptides, US 3 531 258; Brunfeldt K, Reopstorff P and Halstrom J, Reactions System, US 3 557 077; Kubodera T, Hara T and Makabe H, Apparatus for Synthesis of Peptides or the like Organic Compounds, US 3 647 390; Won Kil Park and Regoli D, System for the Solid Phase Synthesis US 3 715 190; Bridgham J et al, Automated Polypeptide Synthesis Apparatus, US 4 668 476; Saneii H H, Solid Phase Synthetizer, US 4 746 490), upto techniques suitable for a parallel synthesis of many peptides (Verlander M S, Fuller W D and Goodman M Rapid, Large Scale, Automable High Pressure Peptide Synthesis, US 4 192 798; Neimark J and Briand J P, Semi-automatic, Solid-phase Peptide Multi-synthetizer and Process for the Production of Synthetic Peptides by the use of Multi-Synthetizer, US 4 748 002; Houghten R A, Means for Sequential Solid Phase Organic Synthesis and Methods using the same, EP 0 196 174; Geysen H M, Meloen R H, and Barteling S J, Proc Nat Acad Sci USA 81, 3998, 1984; Frank R and Doring R, Tetrahedron 44, 6031, 1988; Eichler J, Beyermann M, Bienert M, Collect Czech Chem Commun 54, 1746, 1989; Krchnák V, Vágner J and Mach O, Int J Pept Protein Res 33, 209, 1989). The application of planar continuous carriers made it possible to carry out the so-called continuous synthesis of peptides (Lebl M, Gut V, Eichler J, Krchnák V, Vágner J and Stepánek J, Method of a Continuous Peptide Synthesis on a Solid Carrier, Czechoslovak patent application PV 1280-89).

The present development of molecular biology requires the preparation of many peptides and their anchoring onto various carriers which enable their application in many immunological tests. Hitherto described methods for the multiple synthesis of peptides are not suitable for automation (Houghten R A, Means for Sequential Solid Phase Organic Synthesis and Methods using the same, EP 0 196 174), or they give only a limited quantity of yield, the quality of which cannot be verified in an analytical way (Geysen H M, Meloen R H and Barteling S J, Proc Natl Acad Sci USA 81, 3998, 1984). A drawback of devices using a carrier in the form of particles resides in the necessity to split off the peptide and its new anchoring for later applications. Another drawback of known methods resides in a high consumption of solvents during the synthesis.

The above mentioned drawbacks are obviated by a method for carrying out a multiple synthesis of peptides on a solid carrier with successive connection of active components onto functional groups anchored on a planar, functionalized, porous carrier and by an apparatus for carrying out this method according to the invention.

The principle of the method resides in that individual activated components are put onto separated carriers, while common synthesis steps of corresponding components of various peptides proceed in all compartments of the carrier at the same time. According to the described method, all liquids and solutions of agents are sucked into the carrier and their removal is carried out by pressing the carrier with a dry porous material or by centrifuging the carrier.

One embodiment of the apparatus comprises a planar carrier divided into discrete compartments and a frame which is parallel to the carrier and is provided with windows filled with inert porous material, the position of the windows of the frame corresponding to the position of the compartments of the planar carrier, the positions of the carrier and frame being mutually adjustable.

Another embodiment of the apparatus comprises a planar carrier in the form of a rotatable disk divided into discrete compartments situated along the circumference of the disk and means for rotating the disk. Over the disk, at a point under which individual compartments enter, a metering head is situated. Above the disk is situated a source of radiation directed into the compartments and a detector of the reflected radiation for monitoring the course of condensation reactions of activated components.

An advantage of the invention resides in an automatic parallel performing of condensation reactions causing an increase of a peptidic chain in individual compartments comprising a planar carrier and in simultaneous washing steps and steps resulting in removing temporary protective groups in all compartments with the planar carrier. An important advantage resides in monitoring the course of the chemical reaction and its computer evaluation, by which the synthesis is considerably shortened and made more effective. Another advantage resides in a considerable decrease of solvent consumption during the synthesis and in the possibility to utilize the peptide bonded on the carrier for further applications.

The invention will now be described, by way of example, with reference to the accompanying diagrammatic drawings, in which:
Figure 1 shows a linear movement apparatus for performing a multiple synthesis of peptides on a planar carrier;
Figure 2, shows a rotary movement apparatus; and
Figure 3 is a block diagram of the rotary apparatus shown in Figure 2.

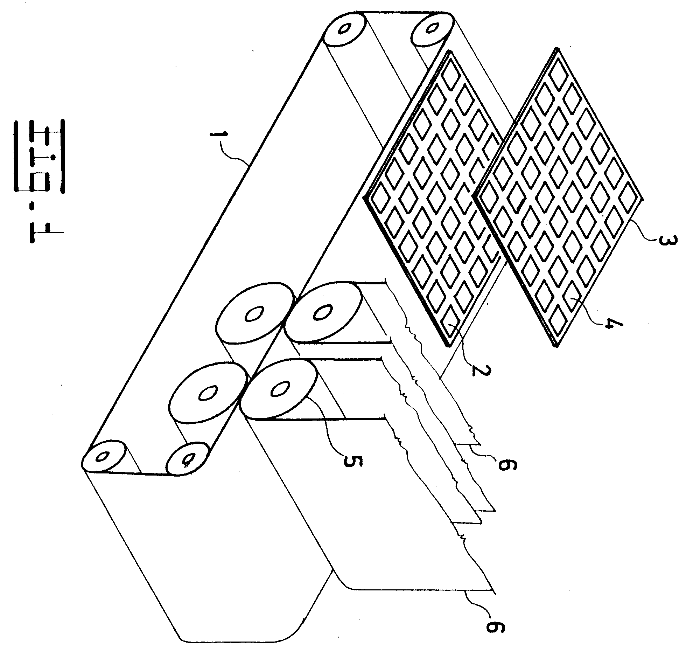

The apparatus according to Figure 1 is formed by a belt 1 made of inert material, eg polyamide or polypropylene, on which is situated a planar carrier divided into compartments 2. A frame 3 comprising windows 4 filled with inert material capable to carry, by means of capillary forces, an agent solution or pure solvent, is situated in such a way that the windows 4 may be aligned with defined compartments 2 of the planar carrier. The apparatus is also provided with holding-down rollers 5, situated one opposite the other, on which a porous dry foil 6 is seated.

By pressing down the frame 3 onto the carrier, transfer of liquid from the windows 4 to the compartments 2 takes place. The material of individual compartments 2 has a higher affinity to the transferred liquid so that major part of the solution is transferred. Glass tissue and cotton seem to be a suitable combination of material for windows 4 and compartments 2. In this case 80% of the liquid (dimethylformamide) was transferred from the window 4 into the compartment 2. The technology of liquid transfer from the window 4 into the compartment 2 ensures simultaneous start of condensation reactions in all parts of the carrier. If it is not necessary to comply with this supposition, it is possible to use the solution of the activated component, as well as solutions used for washing and cleavage of protective groups by micropipettes driven by a stepping motor. The porosity of individual compartments 2 ensures uniform spreading of the used liquid. After having introduced a solution of the activated component, eg symmetrical anhydride amino of a protected amino acid, of respective active ester, or possibly of a mixture of the protected amino acid and activizing agent, advantageously comprising an agent monitoring the condensation course, eg bromophenol blue, then a connection of another amino acid into a peptidic chain takes place. The concentration of an active component must be such that it may be included in the carrier with a sufficient surplus over the present free amino group. Due to the relatively high absorption capacity of cotton (1.0 g of DMF for 1 g of cotton) and relative low substitution applied for the synthesis (0.1 mol/g) of the concentration 0.5 mol/l of activated component it supplies a sufficient surplus securing a quick course of the reaction. After the reaction has been finished, ie after the blue colouring of the carrier has disappeared in case of monitoring with bromophenol blue, liquid is removed from the carrier by passing the carrier together with the porous dry material 6 between the rollers 5.

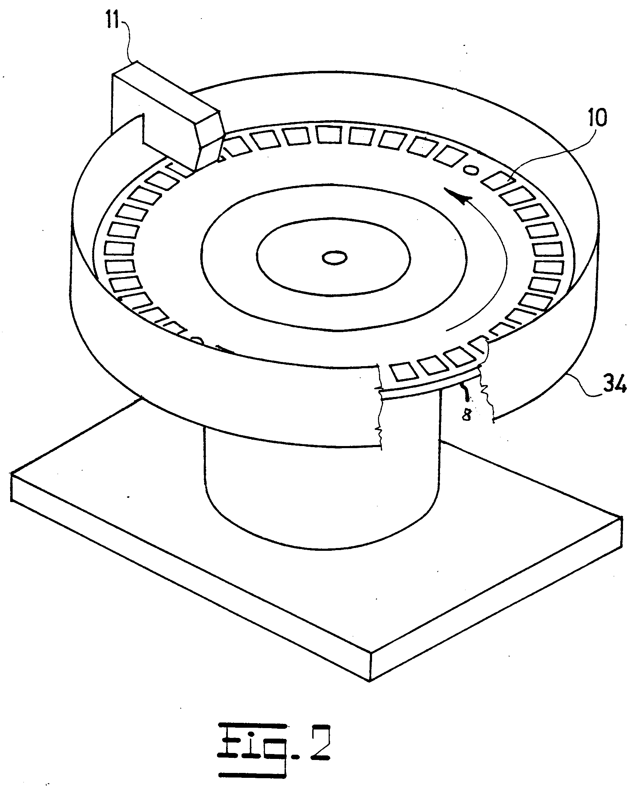

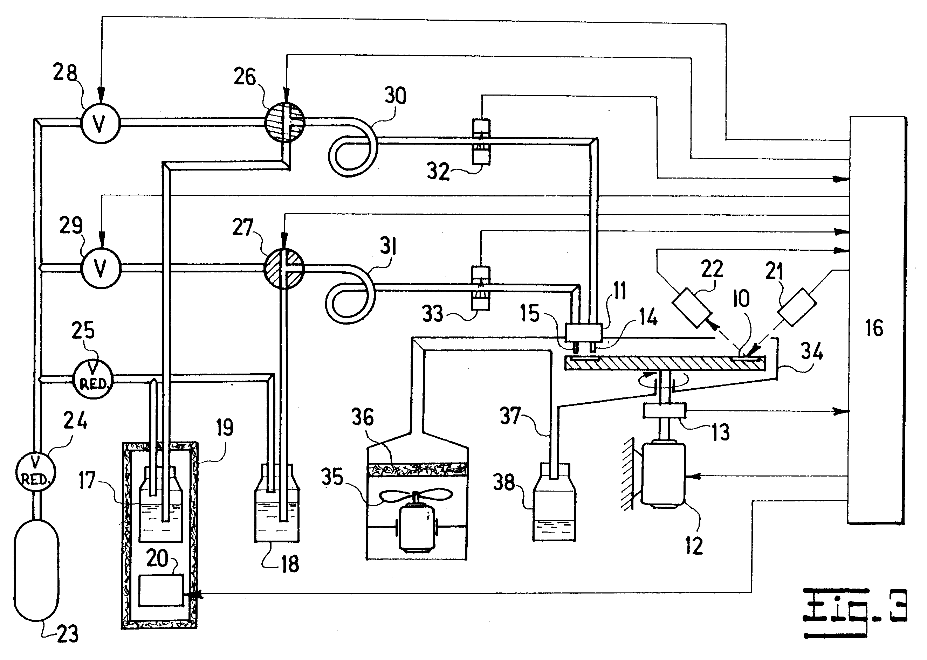

The rotary apparatus according to Figure 2 and Figure 3 comprises a disk 8 made of inert material and provided on its circumference with compartments 10. Over the disk 8, at a point under which the individual compartments 10 enter, a metering head 11 is situated. Above the disk 8 is also situated an optical device comprising a source 21 of light directed into the compartments 10 and a detector 27 of the reflected light. The disk 8 and a rotary incremental position pick-up 13 are seated on the shaft of a driving motor 12. The disk 8 is situated in a tank 34 provided with an exhaust device 35 with a separator 36 and with waste piping 37 which leads into a waste vessel 38. The metering head 11 comprises outlets 14 for activated components and outlets 15 for washing solutions and solutions used for removing the protecting groups. The outlets 14 communicate by means of piping with reservoirs 17 of activated components situated in cooled boxes 19, the temperature of which is controlled by a controller 20. The outlets 15 communicate with reservoirs 18 of washing solutions and solutions used for removing the protecting groups.

The metering system is formed by a container 23 of compressed inert gas, a first and a second pressure reducing valve 24, 25, a first and a second two-way valve 28, 29, a first and a second three-way valve 26, 27, a measuring loop 30 of activated components consisting of a transparent hose and a sensor 32 of the activated component presence and a measuring loop 31 of washing solutions and solutions applied for removing the protecting groups together with a sensor 33 of the presence of the solution. All controlled elements, such as the motor 12, valves and the like, or pick-up elements are connected to a control computer 16.

The number of the outlets 14, 15 of the metering head 11 results from the number of activated components used for the synthesis of peptides and from the number of washing solutions for removing the protecting groups. The metering and transport of activate components and solutions is carried out by the pressure of inert gas. For the process are used two pressure levels controlled by the pressure reducing valves 24, 25. The first pressure reducing valve 24 controls the pressure needed for transporting the measured quantity of liquids into the metering head 11 and from it to the respective compartment 10, the second pressure valve 25 determines optimum velocity of transfer of the measured liquid in the measuring loops 30, 31. The application of activated components and solutions may be carried out also with a higher number of metering heads 11, situated over individual compartments 10 along the circumference of the disk 8. After having supplied the memory of the computer with parameters of the process, of which the most important is the number of sequence of bonded activated components, the synthesis may be started.

The motor 12 turns the disk 8 in such a way that successively into each compartment 10 with a functionalized carrier there may be sprayed, from the reservoir 17, by means of the metering device, the respective activated components. The measuring of the dose of the activated component is carried out as follows: after the respective compartment 10 has assumed its position under the metering head 11, the activated component, after the liquid path between the reservoir 17 and the first measuring loop 30 has been opened by means of the three-way valve 26, is forced out, by the pressure of the inert gas, through the transparent pipe until the sensor 32 of the presence of the activated component is put in function. At this moment the first three-way valve 26 is changed over in such a way that it interconnects the metering loop 30 and the pressure gas inlet and, after the necessary delay, the first two-way valve 28 is opened, which, by means of inert gas pressure set up with the pressure reducing valve 24, forces out the measured quantity of the activated component via the respective outlet 14 of the metering head 11 from the measuring loop 30 onto the carrier. By successive turning of the disk 8 under the metering head 11, all the needed hydraulic paths are activated in this way from reservoirs 17 of activated components, until all compartments 10 of the disk are attended. The motor 12 goes on slowly turning the disk 8, and by means of an optical device comprising the light source 21 and the light detector 22 is followed the course of the chemical reaction, in this case condensation, in individual compartments by comparing the colour of active compartments 10 with reference compartment. For observing the course of the reaction with the optical device, the solution of the activated component must be completed with a respective agent, eg bromophenol blue. At the moment when it is found by means of the optical device that in all active compartments 10 the reaction proceeded well, the disk 8 is rotated at such a speed that residuals of unbonded active components may be centrifuged. When the centrifuging is finished, the disk 8 is again turned slowly so that it is possible, by means of the hydraulic path through valves 25 and 27 and measuring loop 31 and sensor 33 of solution presence, to measure and then by means of the valves 24, 29 and 27 to spray the defined quantity of the washing solution through the outlet 15 of the metering head 11 onto all compartments 10 similarly as described above at metering active components. After centrifuging, this step may be repeated several times. Then, in the same way, the application of the solution used for removing the protecting groups, as well as repeated centrifuging, take place. After several steps, when the washing solution is applied and then centrifuged, the synthesis may proceed to the next step in which the further component is bonded in the described way. The sequence of bonded activated components in individual compartments 10 of the disk 8 is determined in this way on the basis of the peptide sequence determined by the computer, and the synthesis velocity depends on the slowest condensation from all simultaneously proceeding condensations.

The interval for bonding individual activated components is limited and if eg in some compartment the bonding was not successful, the application of the same component is repeated in the next cycle, or the synthesis of this peptide does not continue in the following cycles.

Examples of syntheses which do not limit the mentioned technology but illustrate it only, are mentioned below.

### Example 1

A cotton strip (3 x 27 cm) was esterified with Fmoc-Gly as described in Czechoslovak Patent Application PV1280-89 and then the arm HO-CH₂C₆H₄O(CH₂)₃COOH was added to it. The carrier modified in this way was divided into nine parts and three of them were situated on a glass pad. Into each of these parts of the carrier were added 200 »l of a solution comprising Fmoc-Met (Fmoc-Leu, Fmoc-Nle), diisopropylcarbodiimide, hydroxybenzotriazole (all 0.5 M) and dimethylaminopyridine (0.15 M). The putting in was carried out in such a way that solutions were laid on at first into the square of the glass tissue (3 x 3 cm) which was then pressed onto the cotton carrier and in this way the transfer of the liquid into the carrier was carried out. After twelve hours, the parts of the carrier were washed with dimethyl formamide and dichloromethane. The following solutions were added in a stepwise way using the above mentioned technique into above mentioned parts in the quantity of 200 »l in the sequence:
1. dimethylformamide (3 x 1 min)
2. 20% of piperidine in dimethylformamide (1 x 2 min and 1 x 10 min)
3. dimethylformamide (5 x 1 min)
4. solution of Fmoc-amino acid, N-hydroxybenzotriazole and diisopropylcarbodiimide (all 0.5 M in dimethylformamide) and bromophenol blue (0.5 mM in dimethylformamide)
5. dimethylformamide (3 x 1 min)

After the mentioned time of action, solutions were removed by pressing the carrier together with filtering paper and another portion of the solution was laid on. After the laying on of the solution 4, the carriers were getting blue, and the other step was carried out after the carrier had been decoloured. In individual parts of the carrier there were connected in a stepwise way the following derivatives: Fmoc-Phe, Fmoc-Gly-Gly and Fmoc-Tyr(Bu^{t}). In this way three various peptidic sequences were obtained at the same time (Tyr-Gly-Gly-Phe-Met, Tyr-Gly-Gly-Phe-Leu, Tyr-Gly-Gly-Phe-Nle). These peptides, after having been cleaved from the carrier (90% of trifluoroacetic acid, 5% dimethylsulphide, 5% thioanisole, 3 hours at room temperature), were purified by means of HPLC and characterized in a standard way.

### Example 2

A strip of polypropylene modified with a hydroxypropyl group (Milligen Bioresearch, USA) was esterified in the same way as a cotton tissue, and a carrier was obtained of a substitution 0.1 mmol/g (determination by means of a cleavage of Fmoc group). Then the synthesis was carried out in the same way as in Example 1, only with the difference that one put on less solutions (60 »l) with respect to the lower specific weight of this carrier. The same peptides as in Example 1 were prepared on this carrier.

### Example 3

The synthesis of the above mentioned analogues of enkephalin was carried out on a cotton carrier as was mentioned in Example 1, only with the difference that all solutions were laid onto the carrier by means of a micropipette. The quality of the obtained products was identical with the peptide quality yielded in Example 1.

### Example 4

The synthesis of the above mentioned analogues of enkephalin was carried out on a cotton carrier as was mentioned in Example 1, only with the difference that the compartmentized carrier was connected to the disk circumference and all solutions were removed from the carrier by centrifuging. The quality of the obtained products was identical with the quality of peptides yielded in Example 1.

### Example 5

Six square pieces of cotton (3 x 3 cm, modified by Fmoc-Gly, substitution 0.09 mmol/g) were placed on the perimeter of a planar rotor (diameter 25 cm) with six shallow compartments (3.2 x 4.5 x 0.2 cm). To the centre of the cotton piece the solutions in the order given in the particular example were added. After given time the rotor was spun for 30 seconds at 2500 rpm and next solution was added.

Typical synthetic protocol for the attachment of one amino acid residue consists of the following steps:

### Cleavage:

S.1 Addition of 20% piperidine in dimethylformamide (0.2 ml)
S.2 Waiting 10 min
S.3 Spinning

### Washing:

W.1 Addition of dimethylformamide (0.4 ml)
W.2 Waiting 1 min
W.3 Spinning

### Coupling:

C.1 Addition of 0.1% solution of bromophenol blue in dimethylformamide spiked with N-hydroxybenzotriazole (80 »l)
C.2 Spinning
C.3 Addition of the solution of activated protected amino acid (0.4 ml)
C.4 Waiting until the blue colour of the dot formed in step C.1 disappears (5-120 min)
C.5 Spinning

### Example 6

### Synthesis of Acyl Carrier Protein 65-74

In the first step of the synthesis performed according to the Example 5 Fmoc-Gly-OCH₂C₆H₄OCH₂CH₂CH₂COOH was coupled to the cotton pieces in all six compartments. In the next steps the following amino acid derivatives were coupled to the modified carrier: Fmoc-Asn-OH, Fmoc-Ile-OH, Fmoc-Tyr(Bu^{t})-OH, Fmoc-Asp(OBu^{t})-OH, Fmoc-Ile-OH, Fmoc-Ala-OH, Fmoc-Ala-OH, Fmoc-Glu(OBu^{t})-OH, Fmoc-Val-OH.

The protected amino acid (0.08 mmol) was dissolved in dimethylformamide (0.4 ml) together with N-hydroxybenzotriazole (0.08 mmol) and diisopropylcarbodiimide (0.08 mmol) was added. After 2 minutes the solution was added to the carrier. In the synthesis the following protocol was used:
Cleavage
Washing (3x)
Coupling
Washing (3x)
In the step S.1 (see Example 5) of cleavage various concentrations of piperidine and cleavage times in particular cotton pieces were used:
Compartment
1 - 20% piperidine, 5 min
2 - 20% piperidine, 10 min
3 - 20% piperidine, 20 min
4 - 50% piperidine, 2 min
5 - 50% piperidine, 5 min
6 - 50% piperidine, 10 min
(Cleavage was started at different times so that it could be terminated in all compartments simultaneously by spinning.) At the end of the synthesis the compartments were washed by ethanol and dried. The peptides were cleaved by 50% trifluoroacetic acid, 2% anisole (1h at room temperature), solution was evaporated in vacuo, dissolved in 3M acetic acid and lyophilized. The crude material was analyzed by HPLC (Vydac C18, 25 x 0.4 cm, gradient 20-100% methanol in 0.05% trifluoroacetic acid in 40 min). The quality of peptides synthesized in compartments 4-6 was slightly worse than that from compartments 1-3. Optimum result was obtained from compartment 1. The product was characterized by amino acid analysis (Asp 2.05, Glu 1.04, Gly 1.14, Ala 2.03, Val 0.91, Ile 1.97, Tyr 0.85) and FAB Mass spectroscopy (M+H⁺ = 1064; theory 1064).

### Example 7

### Synthesis of [Ser^{5.}¹⁵]MCH

In the first step of the synthesis performed according to Example 5 N-Fmoc-4-methoxy-4′-(3-carboxypropyloxy)-benzyhydrylamine was coupled to the cotton pieces in all six compartments. In the next steps the following amino acid derivatives were coupled to the modified carrier: Fmoc-Asp(OBu^{t})-OH, Fmoc-Thr(Bu^{t})-OH, Fmoc-Met-OH, Fmoc-Arg(Mtr)-OH, Fmoc-Ser(Bu^{t})-OH, Fmoc-Met-OH, Fmoc-Val-OH, Fmoc-Gly-OH, Fmoc-Arg(Mtr)-OH, Fmoc-Val-OH, Fmoc-Tyr(Bu^{t})-OH, Fmoc-Arg(Mtr)-OH, Fmoc-Pro-OH, Fmoc-Ser(Bu^{t})-OH, Fmoc-Trp-OH, Fmoc-Glu(OBu^{t})-OH, Fmoc-Val-OH.

The synthesis was performed in the same way as in Example 6 with the exception of the step S.1 where different bases were used for the cleavage of the Fmoc protecting group:
Compartment
1 - 20% piperidine, 10 min
2 - 2M 4-benzylpiperidine, 10 min
3 - 0.05M 4-piperidinopiperidine, 10 min
4 - 0.5M 4-(aminomethyl)piperidine, 10 min
5 - 0.5M tris(2-aminoethyl)amine, 10 min
6 - 1M 1-(2-aminoethyl)piperazine, 10 min
The finished peptides were cleaved and analyzed in the same manner as in Example 6. The peptides from compartments 1 and 2 were indistinguishable, other bases afforded a product of inferior quality. Amino acid analysis: Asp 1.09, Thr 1.00, Ser 1.94, Glu 1.10, Pro 1.06, Val 3.25, Met 1.78, Tyr 0.91, Arg 2.85, FAB mass spectrum: 2069.

### Example 8

### Synthesis of Acyl Carrier protein 65-74

The synthesis was performed in the same way as in Example 6. The base used for the cleavage was 20% piperidine in dimethylformamide. In particular cotton pieces the different protocol (number of washing) was applied:
Compartment
1 - Cleavage, Washing (1x), Coupling, Washing (1x)
2 - Cleavage, Washing (2x), Coupling, Washing (2x)
3 - Cleavage, Washing (4x), Coupling, Washing (4x)
4 - The same protocol as in compartment 3, but the modification of the cotton was performed by periodate oxidation and hexamethylenediamine treatment
5 - Cleavage, Washing (1x), Coupling, Washing (1x)
6 - Cleavage, washing (4x), Coupling, Washing (4x)
In compartments 5 and 6 the solution of protected amino acid (0.08 mmol) and HOBt (0.08 mmol) in 0.2 ml dimethylformamide was added to the carrier separately from the 0.4 M solution of diisopropylcarbodiimide in dimethylformamide (0.2 ml).

After the cleavage and analysis performed in the same way as in Example 6 all peptides were found indistinguishable.

### Example 9

### Synthesis of model peptides

In the first step of the synthesis acid-labile amide linker (N-Fmoc-4-methoxy-4'-(3-carboxypropyloxy)benzhydrylamine) was coupled to the cotton squares in compartments 1 to 5. The synthesis was performed in the same manner as in Example 6, but the different sequence was assembled in all compartments:

Compartment 1 - Ala-Val-Leu-Gly-His-Asp-Glu-Ala-
Ala-Tyr-Ser-Lys-Asn-Arg-Arg-Ala-
Val
2 - Asp-Thr-Met-Arg-Ser-Met-Val-Gly-
Arg-Val-Tyr-Arg-Pro-Ser-Trp-Glu-
Val
3 - Tyr-Ala-Ala-Ala-Ala-Ala-Ala-Ala-
Ala-Ala-Ala-Ala-Ala-Ala-Ala-Ala-
Ala-Ala-Ala-Ala-Ala-Val
4 - Tyr-Ala-Ala-Ala-Ala-Ala-Ala-Ala-
Ala-Ala-D-Ala-Ala-Ala-Ala-Ala-
Ala-Ala-Ala-Ala-Ala-Ala-Val
5 - Tyr-Ala-Ala-Ala-Ala-Ala-Ala-D-
Ala-Ala-Ala-Ala-Ala-Ala-Ala-D-
Ala-Ala-Ala-Ala-Ala-Ala-Ala-Val
6: Ala-Ala-Ala-Ala-Ala-Ala-Ala-Ala-
Ala-Ala-Val-Gly

Peptides from the cotton carrier in compartments 1 to 5 were cleaved by trifluoroacetic acid - phenol - water - thioanisole - ethanedithiol (82.5:5:5:5:2.5) mixture (1h, rt) and worked up and characterized in the way described in Example 6. Cotton from compartment 6 was treated with 1M NaOH for 1h, washed and extracted by trifluoroacetic acid. This extract was worked up in the usual way. All products were found more than 80% pure by HPLC. They had correct amino acid analysis and FAB mass spectrum.

### Example 10

Polystyrene resin (153 mg, 1% divinylbenzene, 300-400 mesh) was placed in the "tea bag" according to EP 0 196 174 (Houghten R A) and dimethylformamide was soaked into it. The cotton piece 3 x 3 cm (160 mg) was soaked with dimethylformamide too. The content of solvent in the carrier was determined by weighing. Both carriers were centrifuged (2000 rpm, 2 min) and the content of solvent was determined again. Results of the experiment, together with the attempt to eliminate the liquid from the cotton by its compression together with the dry filtration paper are given in the Table 1.

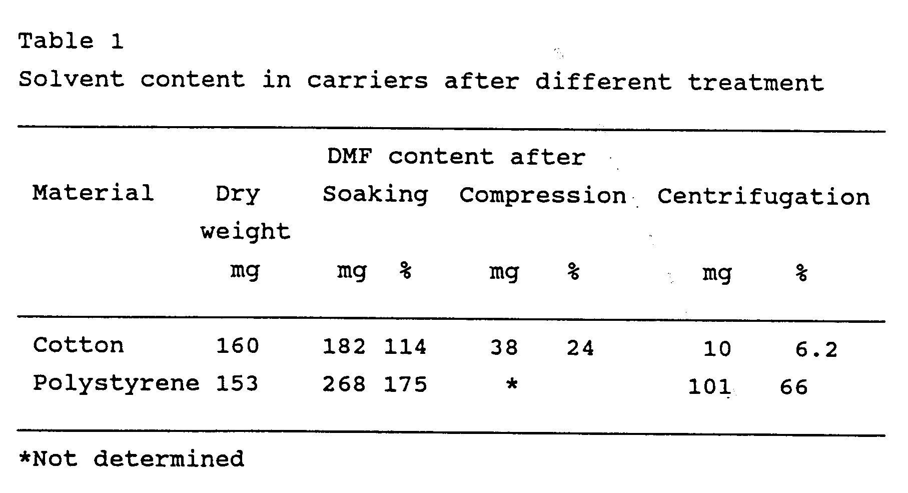

## Claims

1. A method for performing multiple synthesis of peptides on a solid carrier with successive bonding of active components onto functional groups anchored on a planar functionalized porous carrier, wherein individual activized components are put into compartments in the solid carrier, and common steps of the synthesis of various peptides proceed in all compartments of the carrier at the same time, characterized in that all liquids and solutions of agents are absorbed into the carrier and their removing is carried out by pressing the carrier together with dry porous material or centrifuging the carrier.

2. An apparatus for carrying out the method according to Claim 1, characterized in that it comprises a planar carrier divided into discrete compartments (2) and a frame (3) extending parallel to the carrier, the frame being provided with windows (4) filled with inert porous material, the position of the windows of the frame corresponding to the position of the compartments of the planar carrier, the positions of the carrier and of the frame being mutually adjustable.

3. An apparatus for carrying out the method according to Claim 1, characterized in that it comprises a planar carrier in the form of a rotatable disk (8) divided into discrete compartments (10) situated along the circumference of the disk, means (12) for rotating the disk, at least one metering head (11) situated over the disk, at a position below which the compartments enter, the metering head being connected to a measuring system, and an optical device situated above the disk comprising a source of radiation directed into the compartments and a detector of the reflected radiation for monitoring the course of condensation reactions of activated components.

4. An apparatus according to Claim 2 or 3, characterized in that the carrier is represented by cotton or functionalized polypropylene.

## Patentansprüche

1. Verfahren zum Ausführen einer Mehrfachsynthese von Peptiden auf einem festen Träger, bei dem aktive Komponenten mit an einem flachen, funktionalisierten, porösen Träger haftenden funktionellen Gruppen verbunden werden, bei dem einzelne aktivierte Komponenten in Felder auf dem festen Träger gebracht werden und bei dem gemeinsame Verfahrensschritte der Synthese der verschiedenen Peptide in allen Feldern des Trägers gleichzeitig ablaufen, **dadurch gekennzeichnet**, daß alle Flüssigkeiten und Lösungen der Wirkmittel in den Träger absorbiert werden und daß deren Entfernen durch Zusammendrücken des Trägers mit einem trockenen, porösen Material oder durch Zentrifugieren des Trägers ausgeführt wird.

2. Vorrichtung zum Ausführen des Verfahrens nach Anspruch 1, dadurch gekennzeichnet, daß diese einen flachen, in abgegrenzte Felder (2) unterteilten Träger und einen sich parallel zum Träger erstreckenden Rahmen (3) aufweist, wobei der Rahmen mit Fenstern (4), die mit inertem, porösem Material gefüllt sind, versehen ist, wobei der Ort der Fenster des Rahmens dem Ort der Felder des flachen Trägers entspricht und wobei die Orte des Trägers und des Rahmens aufeinander einstellbar sind.

3. Vorrichtung zum Ausführen der Methode nach Anspruch 1, dadurch gekennzeichnet, daß diese einen flachen Träger in der Gestalt einer drehbaren Scheibe (8), die in abgegrenzte, entlang dem Umfang der Scheibe angeordnete Felder (10) unterteilt ist, Mittel (12) zum Drehen der Scheibe, wenigstens einen über der Scheibe an einem Ort, unter dem die Felder eintreten, angeordneten, an eine Meßvorrichtung angeschlossenen Meßkopf (11) und ein über der Scheibe angeordnetes optisches Gerät aufweist, das eine Quelle für auf die Felder ausgerichtete Strahlung und einen Detektor für die zurückgeworfene Strahlung umfaßt, um den Verlauf der Kondensationsreaktionen der aktivierten Komponenten zu überwachen.

4. Vorrichtung nach Anspruch 2 oder 3, dadurch gekennzeichnet, daß Baumwolle oder funktionalisiertes Polypropylen den Träger darstellen.

## Revendications

1. Procédé pour réaliser une synthèse multiple de peptides sur un support solide avec liaison successive de composants activés à des groupes fonctionnels ancrés sur un support poreux plan fonctionnalisé, dans lequel des composants individuels activés sont placés dans des compartiments prévus dans le support solide et les étapes courantes de synthèse de différents peptides sont mises en oeuvre en même temps dans tous les compartiments du support, caractérisé en ce que tous les liquides et les solutions d'agents sont absorbés dans le support, et leur extraction est obtenue en pressant le support avec un matériau poreux sec ou en centrifugeant le support.

2. Appareil pour mettre en oeuvre le procédé selon la revendication 1, caractérisé en ce qu'il comprend un support plan divisé en compartiments discrets (2) et un cadre (3) s'étendant parallèlement au support, ledit cadre présentant des fenêtres (4) remplies d'un matériau poreux inerte, la position des fenêtres du cadre correspondant à la position des compartiments du support plan, et les positions du support et du cadre étant réglables l'une par rapport à l'autre.

3. Appareil pour mettre en oeuvre le procédé selon la revendication 1, caractérisé en ce qu'il comprend un support plan ayant la forme d'un disque tournant (8) divisé en compartiments discrets (10) disposés le long de la circonférence du disque, des moyens (12) pour mouvoir le disque en rotation, au moins une tête de mesure (11) disposée au-dessus du disque à une position au-dessous de laquelle les compartiments arrivent, la tête de mesure étant connectée à un système de mesure, ainsi qu'un dispositif optique disposé au-dessus du disque et comprenant une source de rayonnement dirigée vers l'intérieur des compartiments et un détecteur du rayonnement réfléchi pour surveiller la progression des réactions de condensation des composants activés.

4. Appareil selon la revendication 2 ou la revendication 3, caractérisé en ce que le support est formé de coton ou de polypropylène fonctionnalisé.
